# EUROPEAN PATENT APPLICATION

(11) **EP 4 223 923 A1**
(43) Date of publication of application: **09.08.2023**
(21) Application number: 21875320.0
(22) Date of filing: 21.09.2021
(51) Int. Cl.: D06M 11/74, D06M 15/263, D06M 15/564, A61B 5/263, A61B 5/266

(54) **BIOELECTRODE**

(30) Priority: 29.09.2020 JP 2020163369
(71) Applicant: Toray Industries, Inc., Tokyo 103-8666 (JP)
(72) Inventor: KAMEMOTO, Chigusa, Otsu-shi, Shiga 520-2141 (JP); NAGAI, Noriko, Otsu-shi, Shiga 520-2141 (JP); TONOMORI, Keiichi, Otsu-shi, Shiga 520-2141 (JP)
(74) Representative: Kador & Partner Part mbB
(86) International application number: PCT/JP2021/034536
(87) International publication number: WO 2022/071018

(57) **Abstract**

To obtain a textile-shaped bioelectrode that is flexible, highly comfortable to wear, and unlikely to move out of position, there is provided a bioelectrode having a layered structure of a fiber base layer composed of non-conductive fibers and a conductive layer, wherein the conductive layer is a layer formed of a conductive material including carbon black, urethane resin, and a water-based thickener.

## Description

### TECHNICAL FIELD

The present invention relates to a bioelectrode.

### BACKGROUND ART

Various types of bioelectrodes, such as gel electrodes, rubber electrodes, electrodes using thin metal plates, electrodes using conductive fiber materials, and the like have been used as bioelectrodes for measuring bioelectric signals, such as brain waves, electrocardiograms, electromyograms, and the like of human bodies or animals, or for applying electric stimulation to living bodies.

While the above gel electrodes and rubber electrodes have the advantages of being highly flexible, adherable to a body surface of a living body, and able to stably acquire biosignals, they have a problem in that they have poor breathability, which causes a place of contact being stuffy and develop a rash or the like.

In addition, since the electrode using a thin metal plate is hydrophobic and stiff, it has a problem in that it has little suitability for applications in which the electrode comes into contact with a body surface of a living body, which is moisture-rich and flexible. In order to adhere the electrode to the body surface, a high contact pressure is necessary, or it is necessary to use a conductive paste (jelly).

A textile-shaped electrode having conductivity is considered to be effective as an electrode that can be directly attached to a body surface of a living body without using a conductive paste or the like, and various such proposals have been made. The textile electrode is resistant to bending and can change its shape in accordance with the unevenness of the body surface.

Metal-plated fibers, a conductive polymer coating, and the use of a carbon material have been proposed in order to impart conductivity in a textile-shaped electrode. Carbon is widely used in industrial applications as it is less likely to cause allergic reactions and metal corrosion, while also being inexpensive.

An electrode in which carbon black powder is added to silicon rubber has been proposed as a textile electrode using carbon (see Patent Documents 1 and 2).

### PRIOR ART DOCUMENTS

### PATENT DOCUMENTS

Patent Document 1: Japanese Patent Laid-open Publication No. 2006-512128
Patent Document 2: International Publication No. 2017/199026

### SUMMARY OF THE INVENTION

### PROBLEMS TO BE SOLVED BY THE INVENTION

Both the bioelectrode described in Patent Document 1 and the bioelectrode described in Patent Document 2 have a base material composed of a conductive yarn such as stainless steel or the like or a base material composed of a conductive wire. In addition, the use of silicone rubber (silicone rubber) is exemplified for these bioelectrodes. The base material composed of a conductive yarn such as stainless steel or the like and the base material composed of a conductive wire tend to have poor flexibility compared with a base material composed of non-conductive fibers. In addition, the silicone material included in these bioelectrodes has interfacial characteristics excellent in releasability. Therefore, these bioelectrodes are likely to separate from the skin during use and the bioelectrodes may move out of position due to the movement of a wearer when acquiring biosignals, and there is a concern that artifacts may be generated in the bioelectrodes that acquire or input weak signals. On the other hand, it may not be possible to acquire sufficient conductivity when non-conductive fibers are used.

In view of the above, a problem of the present invention is to provide a textile-shaped bioelectrode, the bioelectrode being flexible, highly comfortable to wear, and unlikely to move out of position.

### SOLUTIONS TO THE PROBLEMS

The configuration of the invention for solving the above problem and achieving the object is any one of the following.
(1) A bioelectrode having a layered structure of a fiber base layer composed of non-conductive fibers and a conductive layer, wherein the conductive layer is formed of a conductive material comprising carbon black, urethane resin, and a water-based thickener.
(2) The bioelectrode according to (1), further comprising a mixed layer in which fibers and a conductive material are mixed located between the fiber base layer and the conductive layer.
(3) The bioelectrode according to (2), wherein a percentage of the mixed layer, which is a value obtained by dividing a thickness value of the mixed layer by a total value of each thickness of the conductive layer, the mixed layer, and the fiber base layer provided in the bioelectrode and multiplying a resultant value by 100, is 0.1 to 80%, and a thickness of the conductive layer is 1 to 200 µm.
(4) The bioelectrode according to any one of (1) to (3), wherein the water-based thickener is a polyacrylic acid-based compound.
(5) The bioelectrode according to any one of (1) to (4), wherein the urethane resin includes at least one selected from a group consisting of an ether-based urethane resin and a carbonate-based urethane resin.
(6) The bioelectrode according to any one of (1) to (5), wherein a volume resistivity of the bioelectrode is 1 × 10⁶ Ω·cm or less.
(7) The bioelectrode according to any one of (1) to (6), wherein a surface resistance value of the fiber base layer is 1 × 10¹⁰ Q or more.

### EFFECTS OF THE INVENTION

The present invention provides a textile-shaped bioelectrode that is flexible, highly comfortable to wear, and unlikely to move out of position.

### BRIEF DESCRIPTION OF THE DRAWINGS

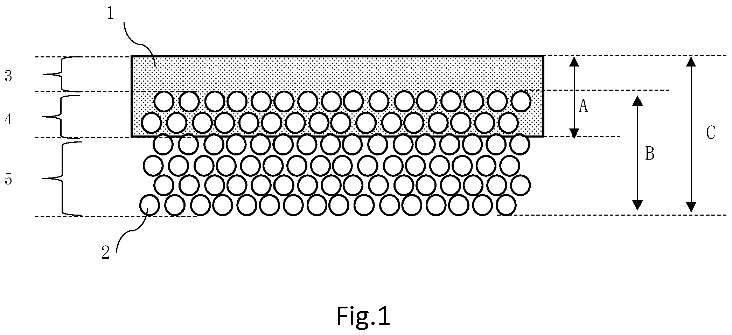

Fig. 1 is a conceptual diagram illustrating a cross section of a bioelectrode showing an embodiment of the present invention.

### EMBODIMENTS OF THE INVENTION

Hereinafter, an embodiment of a bioelectrode according to the present invention will be described in detail. Note that the present invention is not limited to the embodiment.

### <Bioelectrode>

The bioelectrode of the present invention has a layered structure of a fiber base layer composed of non-conductive fibers and a conductive layer, in which the conductive layer serves as a contacting surface with a living body to acquire and transmit biosignals. Here, the above layered structure may be a layered structure formed by directly laminating the fiber base layer and the conductive layer, or it may be a layered structure of 3 or more layers with a mixed layer in which fibers are mixed with a conductive material or the like provided between the fiber base layer and the conductive layer. The conductive layer is a layer formed of a conductive material comprising carbon black, urethane resin, and a water-based thickener, and the mixed layer is a layer in which fibers are mixed with a conductive material. In addition, the mixed layer is formed by impregnating non-conductive fibers with the conductive material. Fig. 1 is a conceptual diagram illustrating a cross section of one mode of the bioelectrode according to the present invention; it shows, for example, an aspect achieved by applying a conductive material 1 on a fiber base material 2, in which the bioelectrode has a structure wherein a conductive layer 3 composed only of a conductive material (i.e., it does not include the fiber base material), a mixed layer 4 including the conductive material and the fiber base material, and a fiber base layer 5 composed only of the fiber base material (i.e., it does not include the conductive material) are layered.

### <Conductive Layer>

In the present invention, the conductive layer is a layer that exhibits conductivity, with the layer formed only of the conductive material (i.e., it does not include the fiber base material). The conductive material as used herein is a mixture for imparting conductivity to the bioelectrode, the mixture comprising (A) carbon black, (B) urethane resin, and (C) a water-based thickener. Also, the mixture and the conductive layer may comprise other components in addition to (A) carbon black, (B) urethane resin, and (C) the water-based thickener. Examples of the other components include, but are not limited to, (D) a conductivity improver, (E) a flexibility imparting agent, (F) a surfactant and/or a leveling agent, a crosslinker, a catalyst, an antifoaming agent, or the like.

### <(A) Carbon Black>

The carbon black (hereinafter, may be referred to as CB) used in the present invention is a conductive substance. When imparting conductivity by CB, it is crucial to consider a diameter of particles forming a CB base (a particle diameter of particles constituting the structure; hereinafter, the particles may be referred to as primary particles and the particle diameter may be referred to as a primary particle diameter), a structure, which is a chain structure of the primary particles, and particle surface properties. In the case of preparing a solution comprising CB and a binder, it is particularly important to increase the specific surface area by modifying the particle surface properties. A large specific surface area means that there are many pores on a particle surface; since the binder enters the pores, a distance between the particles can be reduced, which leads to achieving high conductivity.

The specific surface area can be measured by a BET method, and the BET specific surface area of the carbon black is preferably 400 to 2000 m²/g or more. The BET specific surface area of the carbon black is more preferably 600 to 1600 m²/g. When the BET specific surface area of the carbon black is 400 m²/g or more, the distance between the particles becomes sufficiently small and is in a range in which sufficient conductivity can be imparted to the bioelectrode; when it is 600 m²/g or more, an even higher level of conductive performance can be achieved. When an upper limit is 2000 m²/g or less, the structure can be maintained, and stable conductive performance can be imparted to the bioelectrode. This effect becomes more remarkable when the BET specific surface area of the carbon black is 1600 m²/g or less.

In order to achieve high conductivity, it is desirable that the primary particles of CB are small. An average particle diameter of the primary particles of CB is preferably in the range of 1 to 200 nm, and more preferably in the range of 5 to 100 nm. When the average particle diameter of the primary particles of CB is 1 nm or more, robustness of the bioelectrode against friction becomes apparent; when the average particle diameter is 5 nm or more, a high level of robustness is achieved. When the average particle diameter of the primary particles of CB is 200 nm or less, sufficient conductive performance is imparted to the bioelectrode; further, when the average particle diameter of the primary particles of CB is 100 nm or less, high conductivity is imparted. The primary particle diameter is obtained by measuring diameters of circles circumscribing a plurality of primary particles arbitrarily selected from an image obtained by observing an ultrathin section cut off from a conductive material by a transmission electron microscope (TEM) at an arbitrary magnification at which the primary particle diameter can be observed, then averaging those measured values.

In order to achieve high conductivity, it is desirable that the structure is highly developed, and a dibutyl phthalate (DBP) absorption amount, which is an indicator of structure size, is preferably 100 to 600 cm³/100 g, and more preferably 300 to 500 cm³/100 g. When the dibutyl phthalate (DBP) absorption amount is 100 cm³/100 g or more, the conductive performance becomes apparent due to the development of the structure; when the absorption amount is 300 cm³/100 g or more, the conductive performance is further improved. On the other hand, when the absorption amount is 600 cm³/100 g or less, viscosity can be highly suppressed and sufficient permeation to inside the fibers can be achieved, therefore sufficient conductive performance can be imparted to the bioelectrode. When the absorption amount is 500 cm³/100 g or less, the effect becomes more remarkable. The DBP absorption amount is measured by the method described in JIS K 6217-4(2017) using an absorptometer.

In the bioelectrode of the present invention, a content of (A) carbon black is not particularly limited, but a content of CB is preferably 1 part by weight to 60 parts by weight, and more preferably 5 parts by weight to 40 parts by weight, per 100 parts by weight of a solid content of the conductive material. When the content of CB is 1 part by weight or more, the conductive performance of the bioelectrode becomes apparent due to the continuity of the carbon black; when the content is 5 parts by weight or more, the conductive performance is further improved. When the content of CB is 60 parts by weight or less, or further, 40 parts by weight or less, robustness of the conductive layer against friction can be achieved.

### <(B) Urethane Resin>

The conductive material further comprises (B) urethane resin in addition to (A) the carbon black above. Here, (B) urethane resin plays a role of a binder for supporting the mixture constituting the conductive material on the fiber base material. Since the binder significantly contributes to the flexibility of the bioelectrode, it is important that the binder is urethane resin. Since urethane resin has excellent flexibility compared with resins other than urethane resin, the bioelectrode of the present invention is flexible and highly comfortable to wear.

(B) urethane resin can prevent the mixture constituting the conductive material in the bioelectrode from falling off from the fiber base material, and further, reduce separation of the bioelectrode from the skin and improve biosignal acquisition. From this perspective, one of the characteristics of the present invention is that the conductive material comprises urethane resin.

The conductive material may include, as a binder, a urethane resin alone or 1 or a plurality of other resins; preferable specific examples of the other resins include olefin-based resin, polyester-based resin, urethane resin, epoxy resin, silicone resin, vinyl chloride resin, nylon resin, acrylic-based resin, and the like.

Urethane resins are classified into ether-based, ester-based, carbonate-based, modified polyol, ester/carbonate-based, or a polymer or the like obtained by combining them, depending on a raw material polyol; any of these can be used. From the viewpoint of hydrolysis resistance, it is preferable to include at least one selected from a group consisting of ether-based urethane resin and carbonate-based urethane resin in particular.

Examples of commercially available products that can be used as the urethane resin include the following. That is, examples of ether-based urethane resins include "RESAMINE (registered trademark) D-2040" (Dainichiseika) and "SUPERFLEX (registered trademark) E-4800" (DKS), and carbonate-based urethane resins include "EVAPHANOL (registered trademark) HA-107C" (NICCA CHEMICAL), "RESAMINE (registered trademark) D-6300" (Dainichiseika), "RESAMINE (registered trademark) D-6065NP" (Dainichiseika), and "SUPERFLEX (registered trademark) 460" (DKS), and further, polyether/carbonate-based urethane resins include "RESAMINE (registered trademark) D-4080" (Dainichiseika) and "RESAMINE (registered trademark) D-4200" (Dainichiseika), and the like.

Urethane resins include urethane resins derived from aromatic isocyanates and aliphatic isocyanates; from the viewpoint of toxicity and yellowing, urethane resins having aliphatic isocyanates are desirable for use in bioelectrodes that contact the skin and require durability.

In the bioelectrode of the present invention, from the viewpoint of flexibility of the obtained bioelectrode, a glass transition temperature (Tg) of the urethane resin is preferably -60 to ±0°C. The glass transition temperature (Tg) is measured based on the following method. First, a resin solution is poured into a stainless steel box and dried at 60°C for 1 hour, then at 120°C for 2 hours to create a film with a thickness of about 0.3 mm. Using this film, a dynamic viscoelasticity of -100 to 200°C is measured with a viscoelasticity measuring apparatus DMS6100 (manufactured by Seiko Instruments Inc.), and a peak temperature (T°C) of an obtained loss viscoelastic modulus is defined as the glass transition temperature (Tg). Note that measurement is carried out at a temperature rising rate of 5°C/min and a measurement frequency of 1 Hz.

In addition, from the viewpoint of friction resistance of the obtained bioelectrode, a tensile strength of the urethane resin alone is preferably 5 to 50 MPa. When the tensile strength of the urethane resin alone is 5 MPa or more, robustness of the bioelectrode against friction is obtained; when the tensile strength of the urethane resin alone is 50 MPa or less, a flexibility unique to the textile of the bioelectrode can be more reliably maintained.

In the bioelectrode of the present invention, a content of the urethane resin is not particularly limited, but is preferably 40 to 98 parts by weight, and more preferably 60 to 95 parts by weight per 100 parts by weight of the solid content of the conductive material. When the content of the urethane resin is 40 parts by weight or more, (A) the carbon black comprised in the bioelectrode is less likely to fall off. On the other hand, when the content of the urethane resin is 98 parts by weight or less, contacting points between the carbon black in the bioelectrode are ensured and sufficient conductivity can be ensured when the bioelectrode is used as an electrode member, so this is preferable.

### <(C) Water-based Thickener>

The conductive material further comprises (C) a water-based thickener in addition to (A) the carbon black and (B) the urethane resin. Here, the role of (C) the water-based thickener is to impart viscosity characteristics to a solution (coating liquid) formed of a mixture constituting the conductive layer in order to control a coating thickness and a degree of permeation of the coating liquid into the fiber base material when the coating liquid for forming the conductive layer is applied to the fiber base material. Note that by controlling the coating thickness, the thickness of the conductive layer can be controlled and a bioelectrode having excellent conductivity can be obtained. In addition, by controlling the degree of permeation of the coating liquid into the fiber base material, a thickness of the later-described mixed layer can be controlled and a bioelectrode having excellent conductivity can be obtained.

From the viewpoint of reducing VOC during production, (C) the water-based thickener comprises an aqueous solvent. One type of water-based thickener may be used, or two or more types may be used in combination.

Examples of the water-based thickener include the following. Inorganic-based examples include silicates and montmorillonites, and organic-based examples include cellulose-based carboxymethyl cellulose, methyl cellulose, hydroxyethyl cellulose, vinyl-based polyvinyl alcohol, polyvinyl pyrrolidone, polyvinyl benzyl ether copolymer, polyacrylic acid-based polyacrylic acid or polyacrylate, a poly(meth)acrylic acid-(meth)acrylic acid ester copolymer, a crosslinked core carboxylic acid emulsion, a polyurethane-based polyether-modified urethane compound, a hydrophobically modified polyoxyethylene polyurethane copolymer, a urea-based urethane-urea-based compound, and the like.

Among them, polyacrylic acid-based compounds are preferably used because they can impart thixotropic properties to the coating liquid in addition to thickening properties. The coating liquid imparted with thickening and thixotropic properties by the polyacrylic acid-based compound provides excellent characteristics, such as preventing sedimentation during storage and improving workability due to a reduction in viscosity during coating.

In the bioelectrode of the present invention, a content of (C) the water-based thickener is not particularly limited, but is preferably 0.1 to 10 parts by weight, and more preferably 0.5 to 5 parts by weight per 100 parts by weight of the solid content of the conductive material. When the content of the water-based thickener is 0.1 parts by weight or more, it is possible to impart thickening properties and thixotropic properties to the coating liquid that forms the conductive material of the bioelectrode to improve coatability; when the content is 10 parts by weight or less, coating defects due to excessive thickening can be suppressed, so this is preferable.

### <Other Mixtures>

As described above, in the bioelectrode of the present invention, the conductive material may comprise other components in addition to (A) the carbon black, (B) the urethane resin, and (C) the water-based thickener. Examples of the other components include (D) a conductivity improver, (E) a flexibility imparting agent, (F) a surfactant and/or a leveling agent, a crosslinker, a catalyst, an antifoaming agent, or the like.

### <(D) Conductivity Improver>

(D) a conductivity improver may be added to the conductive material. Examples of (D) the conductivity improver include, but are not limited to, for example, compounds having a boiling point of 100°C or higher and two or more hydroxyl groups in the molecule, compounds having a boiling point of 100°C or higher and at least one sulfinyl group in the molecule, compounds having a boiling point of 60°C or higher and at least one carbonyl group in the molecule, compounds having a boiling point of 100°C or higher and at least one amide group in the molecule, and the like. One of these (D) conductivity improvers may be used alone, or two or more may be used in combination.

Examples of compounds having a boiling point of 100°C or higher and two or more hydroxyl groups in the molecule include, for example, ethylene glycol, diethylene glycol, propylene glycol, trimethylene glycol, β-thiodiglycol, triethylene glycol, tripropylene glycol, 1,4-butanediol, 1,5-pentanediol, 1,3-butanediol, 1,6-hexanediol, neopentyl glycol, catechol, cyclohexanediol, cyclohexanedimethanol, glycerin, erythritol, inmatol, lactitol, maltitol, mannitol, sorbitol, xylitol, sucrose, and the like. One of these may be used alone, or two or more may be used in combination.

Examples of compounds having a boiling point of 100°C or higher and at least 1 sulfinyl group in the molecule include, for example, dimethyl sulfoxide and the like.

Examples of compounds having a boiling point of 60°C or higher and at least one carbonyl group in the molecule include, for example, acrylic acid, methacrylic acid, methanoic acid, ethanoic acid, propanoic acid, butanoic acid, pentanoic acid, hexanoic acid, octanoic acid, decanoic acid, dodecanoic acid, benzoic acid, p-toluic acid, p-chlorobenzoic acid, p-nitrobenzoic acid, 1-naphthoic acid, 2-naphthoic acid, phthalic acid, isophthalic acid, oxalic acid, malonic acid, succinic acid, adipic acid, maleic acid, fumaric acid, and the like. One of these may be used alone, or two or more may be used in combination.

Examples of compounds having a boiling point of 100°C or higher and at least 1 amide group in the molecule include, for example, N,N-dimethylacetamide, N-methylformamide, N,N-dimethylformamide, acetamide, N-ethylacetamide, N-phenyl-N-propylacetamide, benzamide, and the like. One of these may be used alone, or two or more may be used in combination.

When the conductive material comprises (D) a conductivity improver, a content thereof is not particularly limited, but is preferably 0.01 to 20 parts by weight, and more preferably 0.1 to 10 parts by weight per 100 parts by weight of the solid content of the conductive material. When the content of (D) the conductivity improver is 0.01 parts by weight or more, an effect of improving conductivity is sufficiently achieved; when the content of (D) the conductivity improver is 20 parts by weight or less, dryability of the bioelectrode is satisfactory.

### <(E) Flexibility Imparting Agent>

(E) a flexibility imparting agent may be added to the conductive material. (E) the flexibility imparting agent is not particularly limited, and examples include, for example, glycerol, sorbitol, polyglycerin, polyethylene glycol, polyethylene glycol-polypropylene glycol copolymer, and the like. One of these may be used alone, or two or more may be used in combination.

When the conductive material comprises (E) the flexibility imparting agent, a content thereof is not particularly limited, but is preferably 0.01 to 20 parts by weight, and more preferably 0.1 to 10 parts by weight per 100 parts by weight of the solid content of the conductive material. When the content of (E) the flexibility imparting agent is 0.01 parts by weight or more, sufficient flexibility is achieved; when the content of (E) the flexibility imparting agent is 20 parts by weight or less, the bioelectrode has excellent conductivity and strength and does not have a significantly reduced resistance to washing.

### <(F) Surfactant and/or Leveling Agent>

In the present invention, a surfactant for uniformly dispersing the mixture constituting the conductive material in the conductive material or a leveling agent for making the surface tension of the bioelectrode uniform during drying may be added to the conductive material. Note that in the bioelectrode of the present invention, one compound may correspond to both the surfactant and the leveling agent. In addition, when the surfactant and the leveling agent are different compounds, the surfactant and the leveling agent may be used in combination.

As the surfactant, those having dispersibility in the solvent of the solid component are preferable. Specific examples thereof include, for example, siloxane-based compounds such as polyether-modified polydimethylsiloxane, polyether-modified siloxane, polyether ester-modified hydroxyl group-containing polydimethylsiloxane, polyether-modified acrylic group-containing polydimethylsiloxane, polyester-modified acrylic group-containing polydimethylsiloxane, perfluoropolydimethylsiloxane, perfluoropolyether-modified polydimethylsiloxane, and perfluoropolyester-modified polydimethylsiloxane; fluorine-containing organic compounds such as perfluoroalkylcarboxylic acids and perfluoroalkylpolyoxyethylene ethanol; polyether-based compounds such as polyoxyethylene alkylphenyl ethers, propylene oxide polymers, and ethylene oxide polymers; carboxylic acids such as coconut oil fatty acid amine salts and gum rosin; ester-based compounds such as castor oil sulfuric acid esters, phosphoric acid esters, alkyl ether sulfates, sorbitan fatty acid esters, sulfonic acid esters, and succinate esters; sulfonate compounds such as alkylarylsulfonate amine salts and dioctyl sodium sulfosuccinate; phosphate compounds such as sodium lauryl phosphate; amide compounds such as coconut oil fatty acid ethanolamide; acrylic-based compounds; and the like. One of these surfactants may be used alone, or two or more may be used in combination.

The leveling agent is not particularly limited. Examples include, for example, siloxane-based compounds such as polyether-modified polydimethylsiloxane, polyether-modified siloxane, polyether ester-modified hydroxyl group-containing polydimethylsiloxane, polyether-modified acrylic group-containing polydimethylsiloxane, polyester-modified acrylic group-containing polydimethylsiloxane, perfluoropolydimethylsiloxane, perfluoropolyether-modified polydimethylsiloxane, and perfluoropolyester-modified polydimethylsiloxane; fluorine-containing organic compounds such as perfluoroalkylcarboxylic acids and perfluoroalkylpolyoxyethylene ethanol; polyether-based compounds such as polyoxyethylene alkylphenyl ethers, propylene oxide polymers, and ethylene oxide polymers; carboxylic acids such as coconut oil fatty acid amine salts and gum rosin; ester-based compounds such as castor oil sulfuric acid esters, phosphoric acid ester, alkyl ether sulfates, sorbitan fatty acid esters, sulfonic acid esters, and succinate esters; sulfonate compounds such as alkylarylsulfonate amine salts and dioctyl sodium sulfosuccinate; phosphate compounds such as sodium lauryl phosphate; amide compounds such as coconut oil fatty acid ethanolamide; and the like. One of these leveling agents may be used alone, or two or more may be used in combination.

### <Fiber base layer>

As the fibers constituting the fiber base layer provided in the bioelectrode of the present invention, non-conductive fibers are used instead of using metal fibers such as stainless steel, aluminum, aluminum alloy, or copper; metal-coated fibers coated with a metal such as silver; conductive fibers spun using a resin kneaded with metal or carbon; or the like. The term "non-conductive" as used herein indicates a surface resistance value of 1 × 10¹⁰ Ω or more. Non-conductive fibers tend to be more easily available compared with conductive fibers, thus the bioelectrode of the present invention is excellent in terms of productivity.

The non-conductive fibers constituting the fiber base layer used in the present invention may be natural fibers or chemical fibers. Examples of natural fibers include cellulose-based fibers such as cotton and hemp, protein fibers such as wool and silk, and the like. Examples of chemical fibers include regenerated fibers such as rayon, semisynthetic fibers such as acetate, synthetic fibers, and the like. From the viewpoint of workability, the fibers constituting the fiber base layer used in the present invention are preferably synthetic fibers.

Examples of the synthetic fibers include polyamide fibers such as nylon and aramid, polyester fibers such as polyethylene terephthalate, acrylic fibers such as polyacrylonitrile, polyolefin fibers such as polyethylene and polypropylene, polyvinyl alcohol fibers, polyvinyl chloride-based fibers, polyurethane fibers, polyimide fibers, and other heterocyclic polymer fibers. From the viewpoint that higher flexibility can be imparted to the bioelectrode, it is preferable to use any one or a plurality of types of fibers among polyamide fibers, polyester fibers, and polyolefin fibers in particular.

In the bioelectrode of the present invention, a cross-sectional shape of the fibers constituting the fiber base layer may be a round cross-section, a triangular cross-section, a flat cross-section, a polygonal cross-section, a hollow shape, or another irregular cross-sectional shape having a high degree of irregularity, and it is not particularly limited.

Examples of a form of the fiber base layer according to the present invention include mesh, paper, woven fabric, knitted fabric, non-woven fabric, ribbon, string, and the like. The form is not particularly limited as long as it is suitable for the purpose of use; from the viewpoint of conductivity and workability, a non-woven fabric or a woven fabric is desirable.

In the case of a non-woven fabric, it is desirable that the fabric has high water pressure resistance. It is more preferable to have a performance of 50 mmH₂O (490 Pa) or more, and further, 100 mmH₂O (980 Pa) or more. As a means for achieving water pressure resistance, a value within the above range can be achieved by setting a layered structure of a spunbond layer and a melt blown layer, by increasing non-woven fabric basis weight, or by performing embossing.

In the case of a woven fabric, it is desirable that a total of each cover factor of a warp yarn and a weft yarn of a woven fabric, in respect to a cover factor calculated by multiplying a square root of a fineness by a yarn density, is 1500 or more and 3000 or less. When the cover factor is small, a coating agent may reach the back surface and the fiber base layer may not function as an insulating layer. On the other hand, a woven fabric having a cover factor exceeding 3000 reduces weavability.

A form of the fibers may be any of monofilament yarns, multifilament yarns, or staple yarns.

A single fiber fineness is not particularly limited and may be, for example, about 0.0001 dtex to 300 dtex.

The fiber base layer is subjected to many treatments such as a shrinkage treatment, a form fixing treatment, a compression treatment, a dyeing finishing treatment, an oil application treatment, a heat fixing treatment, solvent removal, form fixing agent removal, a combing treatment, a polishing treatment, a plane (roll) press treatment, and a high-performance short-cut shearing treatment (cutting raised fibers), in addition to a fiber entangling treatment and a raising treatment, with the treatments appropriately combined in each step; the implementation is not limited as long as the performance as an electrode is not impaired.

In addition, it is preferable that the fiber base layer has a basis weight of 5 g/m² or more and 500 g/m² or less. When the basis weight is 5 g/m² or more, the fabric is not too thin and the bioelectrode has sufficient strength; when the basis weight is 500 g/m² or less, the bioelectrode has satisfactory flexibility. The basis weight is more preferably 10 g/m² or more and 250 g/m² or less.

Further, it is preferable that the fiber base layer has a surface resistance value of 1 × 10¹⁰ Q or more when measuring with the fiber base material alone by a later-described method. When the surface resistance value is 1 × 10¹⁰ Q or more, it is possible to further suppress the occurrence of rigidity in the bioelectrode when the conductive layer is applied; as a result, the bioelectrode is highly comfortable to wear. Note that when a conductive yarn or a conductive wire made of stainless steel or the like is used, a resistance value of the fiber base material alone is reduced, and the surface resistance value tends to be less than 1 × 10¹⁰ Q. In addition, when the surface resistance value is 1 × 10¹⁰ Q or more, that is, when the conductivity of the fiber base material itself is low, it is possible to take greater advantage of the fiber base layer as an insulating layer. Further, examples of a fiber base material having a surface resistance value exceeding 1 × 10¹⁸ Q are those using a fluorine-based polymer. From the viewpoint that the processing agent has more excellent coatability in addition to the viewpoint above, it is preferable that the surface resistance value is 1 × 10¹⁸ Q or less. Note that the surface resistance value of the fiber base material alone can be controlled by the selection of the polymer forming the fiber base material or by limiting the application of the conductive substance during or after forming the base material.

### <Mixed Layer>

In the present invention, the conductive layer is supported on the fiber base layer, and it is preferable that a mixed layer is provided between the conductive layer and the fiber base layer. Here, the mixed layer is a layer in which fibers and a conductive material are mixed, and in which the conductive material is inserted into gaps between the fibers. When a mixed layer is present, adhesion between the conductive layer and the fiber base layer is firm, and separation of layers is unlikely to occur during use. It is preferable that the mixed layer is provided such that a percentage of the mixed layer measured by a later-described method is 80% or less. The percentage of the mixed layer is more preferably 0.1% or more and 80% or less, and further preferably 0.5% or more and 50% or less. Here, as is described later, the percentage of the mixed layer is a value obtained by dividing the thickness value of the mixed layer by the total value of each thickness of the conductive layer, the mixed layer, and the fiber base layer provided in the bioelectrode and multiplying a resultant value by 100.

### <Other Configurations of the Bioelectrode>

In the bioelectrode of the present invention, a resin layer may be layered, or fibers, a film, or the like may be layered, on a surface opposite to the conductive layer which is a contacting surface with a living body. The resin, fibers, or film can be layered for the purpose of insulating against electric stimulation, moisture permeation, moisture retention, and the like.

### <Percentage of the Mixed Layer and Conductive Layer Thickness of the Bioelectrode>

The percentage of the mixed layer and the conductive layer thickness of the bioelectrode of the present invention can be controlled by the selection of the thickener or the mixing amount of the thickener in the coating liquid, in addition to the selection of the polymer constituting the fiber base material, a fineness, and a density. When the percentage of the mixed layer is measured by a measurement method described in the following Examples section, the percentage of the mixed layer is preferably 0.1% or more and 80% or less, and more preferably 0.5% or more and 50% or less.

The percentage of the mixed layer as used herein specifically refers to a value obtained by dividing the thickness value of the mixed layer provided in the bioelectrode by the total value of each thickness of the conductive layer, the mixed layer, and the fiber base layer provided in the bioelectrode and multiplying a resultant value by 100.

If the percentage of the mixed layer is too high, the resistance value of the bioelectrode increases due to the insulating property of the fiber base material, and as a result, conductivity is reduced, which is not preferable. In the bioelectrode of the present invention, when the percentage of the mixed layer is set as 80% or less, it is possible to provide an electrode in which the resistance value is suppressed to be low, and conductivity is more satisfactory. The percentage of the mixed layer is more preferably 50% or less. On the other hand, if the percentage of the mixed layer is low, the conductive layer and the fiber base layer tend to separate, which is not preferable. In the bioelectrode of the present invention, when the percentage of the mixed layer is 0.1% or more, and more preferably 0.5% or more, separation between the conductive layer and the fiber base layer can be suppressed.

In addition, when the thickness of the conductive layer is measured by the measurement method described in the following Examples section, it is desirable that the thickness is 1 to 200 um. If the thickness of the conductive layer is too thin, an influence of the insulating property of the fiber base material gets stronger and the resistance value of the bioelectrode is likely to increase; as a result, conductivity tends to be reduced, which is not preferable. If the thickness of the conductive layer is too thick, the flexibility of the fiber base material is likely to be impaired and rigidity occurs, which may impair the comfortability to wear. In the bioelectrode of the present invention, when the thickness of the conductive layer is set to 1 to 200 pm, it is possible to provide an electrode having a higher level of both conductivity and flexibility.

In addition, from the viewpoint that it is possible to provide an electrode in which the resistance value is suppressed to be low and conductivity is more satisfactory, separation between the conductive layer and the fiber base layer can be suppressed, and further, flexibility is excellent, it is particularly preferable that the percentage of the mixed layer is 0.1 to 80%, and further, the thickness of the conductive layer is 1 to 200 µm.

### <Method for Manufacturing the Bioelectrode>

The bioelectrode of the present invention is obtained, for example, by applying the coating liquid of the conductive material comprising (A) carbon black, (B) urethane resin, and (C) the water-based thickener on a surface of a fiber base layer to form a layered structure of the fiber base layer and the conductive layer. Note that it is preferable to use a dispersion liquid including (A) carbon black, (B) urethane resin, and (C) the water-based thickener, a solution comprising (A) carbon black, (B) urethane resin, and (C) the water-based thickener, or the like as the coating liquid and coat that on the surface of the fiber base layer. Note that in the present specification, a substance that completely dissolves all the components included in the conductive material (i.e., a "solvent") and a substance that disperses the insoluble components (i.e., a "dispersion medium") are both referred to as a "solvent" without any particular distinction. The solvent will be described below.

### <Solvent>

The above solvent is not particularly limited and includes, for example, water; alcohols such as methanol, ethanol, 2-propanol, 1-propanol, and glycerin; ethylene glycols such as ethylene glycol, diethylene glycol, triethylene glycol, and tetraethylene glycol; glycol ethers such as ethylene glycol monomethyl ether, ethylene glycol monoethyl ether, ethylene glycol mono-normal butyl ether, diethylene glycol monomethyl ether, ethylene glycol diethyl ether, and diethylene glycol dimethyl ether; glycol ether acetates such as ethylene glycol monoethyl ether acetate, diethylene glycol monoethyl ether acetate, and diethylene glycol monobutyl ether acetate; propylene glycols such as propylene glycol, dipropylene glycol, and tripropylene glycol; propylene glycol ethers such as propylene glycol monomethyl ether, propylene glycol monoethyl ether, dipropylene glycol monomethyl ether, dipropylene glycol monoethyl ether, propylene glycol dimethyl ether, dipropylene glycol dimethyl ether, propylene glycol diethyl ether, and dipropylene glycol diethyl ether; propylene glycol ether acetates such as propylene glycol monomethyl ether acetate, propylene glycol monoethyl ether acetate, dipropylene glycol monomethyl ether acetate, and dipropylene glycol monoethyl ether acetate; tetrahydrofuran; acetone; acetonitrile; and the like. One of these solvents may be used alone, or two or more may be used in combination.

It is preferable that the solvent is water or a mixture of water and an organic solvent. In the case that water is included as the solvent, a content of water is not particularly limited, but is preferably 10 to 1000 parts by weight, and more preferably 20 to 500 parts by weight per 100 parts by weight of the solid content of the conductive material. When the content of water is 10 parts by weight or more, fluidity of the mixture in the solution is ensured and handling is satisfactory; when the content is 1000 parts by weight or less, a concentration of the mixture does not drop too low and an amount of coating liquid used does not increase too much.

In the present invention, when applying the coating liquid of the conductive material on the fiber base layer, an ordinary method such as a dipping method, a coating method, a spraying method or the like may be used; by laminating the coating liquid on the fiber base material then heating, the bioelectrode can be obtained. From the viewpoint that the coating liquid of the conductive material can be coated in a uniform thickness, the coating method is preferable.

It is desirable that (A) carbon black is mixed with (E) the surfactant in advance and uniformly dispersed in the solvent, and it is desirable that dispersibility is maintained when (B) urethane resin is further mixed. Examples of the method for uniformly dispersing carbon black in a solvent include, for example, a mechanical stirring method using a ball mill, a bead mill, a planetary ball mill, a vibration ball mill, a sand mill, a colloid mill, an attritor, a roll mill, high-speed impeller dispersion, a disperser, a homogenizer, a high-speed impact mill, ultrasonic dispersion, a stirring blade, a stirrer, or the like, but are not limited hereto.

### <Mode for Use of the Bioelectrode>

As a preferred mode for use of the bioelectrode of the present invention, an example includes a form that allows direct contact with a living body to acquire electric signals and/or the application of electric signals or electric stimulation. Examples include electrocardiographic, electromyographic, and electroencephalographic electrode members for acquiring electric signals from a living body, and low-frequency, high-frequency, and EMS electrode members for applying electric stimulation to a living body.

Preferable examples of specific forms are those in direct contact with the skin, and can include electrodes, electric wires, jackets, pants, gloves, socks, brassieres, headbands, wristbands, scarves, hats, belly warmers, supports, shoes, sheets, glasses, headbands, decorative hair slides, headphones, watches, chairs, toilet seats, steering wheels, beds, carpets, various covers, and the like that include the bioelectrode, but are not limited hereto. These various products can be given electrode member functions by attaching a sensor, an electric stimulation device, or the like to a part of these products such that, for example, an electrical connection can be established with the bioelectrode.

In the case of an electrode, a form of the electrode alone and/or in combination with the above forms that are in direct contact with the skin can also be suitably used. The shape of the electrode alone may be any shape, such as circular, polygonal, and the like, and is not limited.

A size of the electrode may be set as a contacting area for enabling the acquisition of desired biosignals, and is not limited. In order to improve adhesion to a living body, a general flat electrode may have a three dimensional structure, such as a loop shape or the like, such that it works easily alongside movement, or it may be inflated with air.

In the case that the electrode is used in combination with another structure, such as clothes and the like, wiring may be joined to the bioelectrode such that the electric signals of a desired site can be acquired; further, a button, a hook, a magnet, a hook-and-loop fastener represented by "MAGICTAPE" (registered trademark), or the like may be joined to the bioelectrode such that the electrode can also be suitably used in a form that is detachable from clothes.

### <Static Friction Resistance of the Bioelectrode>

In the bioelectrode of the present invention, static friction resistance can be controlled by appropriately selecting a binder resin. When the later-described static friction resistance (Fs) is measured, a value of 0.16 N/cm² or more is desirable. When the bioelectrode has little static friction resistance, the bioelectrode may move out of position due to the movement of a wearer when acquiring biosignals, and there is a concern that artifacts may be generated in bioelectrodes that acquire or input weak signals. Since the bioelectrode of the present invention has a large static friction resistance, it is possible to prevent the bioelectrode moving out of position due to the movement of a wearer.

### <Volume Resistivity of the Bioelectrode>

Volume resistivity of the bioelectrode of the present invention can be controlled by the properties of CB (BET specific surface area and amount of DBP deposited), a CB mixing amount in the conductive material, and the percentage of the mixed layer, and the like.

It is preferable that the volume resistivity of the bioelectrode of the present invention is 1 × 10⁶ Ω·cm or less when measured by a later-described method. It is more preferable that the volume resistivity is 1 × 10⁻² Ω·cm or more and 1 × 10⁶ Ω·cm or less. When the volume resistivity is 1 × 10⁶ Ω·cm or less, the bioelectrode is more excellent in acquiring biosignals. On the other hand, when the volume resistivity is 1 × 10⁻² Ω·cm or more, an amount of the binder supporting CB is increased; as a result, a CB holding force in the bioelectrode is improved and CB is further suppressed from falling off the bioelectrode.

### EXAMPLES

Hereinafter, the bioelectrode of the present invention will be described in detail with reference to the examples. The bioelectrode of the present invention is not limited to these examples. The measured values in the examples and comparative examples are obtained by the following methods.

### <Static Friction Resistance of the Bioelectrode>

The static friction resistance of the bioelectrode is measured by the method described below.

Using a surface property measuring machine Tribogear (type: HEIDON-14DR) manufactured by Shinto Scientific Co., Ltd. with a moving speed of 60 mm/min and a load of 255 g (2.50 N), the electrode is fixed to a flat indenter (area of 30 × 28 mm), then an SUS plate with a surface roughness of 0.8 um, which is a friction member, is slid in a perpendicular direction to measure the static friction resistance (N). The static friction resistance force, which is a value measured at three points and averaged, is divided by the surface area of the flat indenter to obtain a static friction resistance force per unit area (N/cm²). Note that the load is set in order to apply a wearing pressure corresponding to the area of the flat indenter.

### <Surface Resistance Value of the Fiber base layer>

The surface resistance value (Ω) of the fiber base layer of the bioelectrode is measured using a surface electric resistance meter (Megaresta H0709 manufactured by SHISHIDO ELECTROSTATIC, LTD.) under an environment of 20°C and 40% RH at 500 V for 60 seconds. The value is measured at three points and averaged.

### <Percentage of the Mixed Layer of the Bioelectrode>

The percentage of the mixed layer of the bioelectrode is measured by the following method. The electrode is cut with a single blade and a cross-sectional image is acquired with a microscope (VHX-2000 manufactured by KEYENCE CORPORATION) at a magnification of 100 times. Using the measurement mode, the thickness (µm) of the conductive layer formed only of the conductive material, the thickness (µm) of the fiber base layer formed only of the fiber base layer, and the thickness (µm) of the mixed layer between the conductive layer and the fiber base layer in which the conductive material and the fibers are mixed are each measured.

A specific method for measuring the thickness of each layer from the cross-sectional image will be described in detail with reference to Fig. 1. Three arbitrary points where the conductive material permeates from the surface side of the conductive layer toward the fiber base layer are selected, a total thickness of the conductive layer and the mixed layer is measured at each point, and an average value thereof is defined as A. In addition, a total thickness of the fiber base layer and the mixed layer is measured at another three arbitrarily selected points where the conductive material penetrates the fiber base material, and an average value thereof is defined as B. Further, a thickness from a surface on the conductive layer side to a surface on the fiber base layer side is measured at another three arbitrarily selected points as the total (bioelectrode thickness) of the conductive layer, the mixed layer, and the fiber base layer, and an average value thereof is defined as C. The thickness of the mixed layer is calculated as (A + B) - C and defined as D. The thickness of the conductive layer is calculated as A - D. The thickness of the fiber base layer is calculated as B - D. In addition, the percentage (%) of the mixed layer is a value obtained by dividing the thickness of the mixed layer by the total value of each thickness of the conductive layer, the mixed layer, and the fiber base layer provided in the bioelectrode, and multiplying a resultant value by 100. Note that in a case where a surface of the base material has unevenness such as embossing, and the layer thickness varies depending on location, an image is acquired at a site in contact with the living body to measure the layer thickness.

### <Resistance Value and Volume Resistivity of the Bioelectrode>

The measurement is performed by a five-point measurement method described in JIS K 7194 (1994). The resistance value (Ω) of the bioelectrode is measured under an environment of 20°C and 40% RH using a resistivity meter (Mitsubishi Chemical Analytech four probe resistance meter Loresta-AX MCP-T370). In addition, the value of the thickness of the bioelectrode used for calculating the volume resistivity is the total value of each thickness of the conductive layer and the mixed layer. The electrode is cut with a single blade and an image is acquired with a microscope (VHX-2000 manufactured by KEYENCE CORPORATION) at a magnification of 100 times, then, using the measurement mode, the thickness (µm) of the conductive layer formed only of the conductive material, and the thickness (µm) of the mixed layer between the conductive layer and the fiber base layer in which the conductive material and the fibers are mixed, are each measured. The volume resistivity (Ω·cm) is calculated from the obtained resistance value, thickness, and a correction coefficient prescribed in JIS.

### <Rate of Bending Resistance Change of the Bioelectrode>

A rate of bending resistance change (times) of the bioelectrode is calculated by measuring each of the bending resistance (mm) of the bioelectrode and the bending resistance (mm) of the fiber base material before conductive processing according to JIS L 1096 (fabric testing method for woven and knitted fabrics) (1999) bending resistance A method (45° cantilever method), then dividing the bending resistance of the bioelectrode by the bending resistance of the fiber base material. Note that a sample for measuring the bending resistance is taken such that a vertical direction or a wale direction is longitudinal, and the measurement is performed on the front and back, then the obtained values are averaged.

### <Peel Strength of the Conductive Layer>

The measurement is carried out using the following method with reference to the peel strength test method of JIS L 1066 (2004). Three 15 × 2.5 cm test pieces are prepared with the vertical direction of the sample set as the longitudinal direction. For each test piece, in a state where exactly 5 cm of the conductive layer is peeled off from the test piece in the longitudinal direction of the test piece and using a low-speed extension type tensile tester with an automatic recording device, a portion of the peeled conductive layer and a portion of the remaining base material are held at a gripping interval of 5 cm, and 5 cm of the conductive layer is further peeled off at a tensile speed of 10 cm/min, with the load (N) at that time then measured. Three maximum values (N) are taken from the larger side, and three minimum values (N) are taken from the smaller side of a load-peeling distance curve, and an average value thereof is calculated. This is carried out on each test piece, and further, an average is calculated as the peel strength. A case in which the peel strength is 30 cN/cm or more is defined as A, and a case in which the peel strength is less than 30 cN/cm is defined as B; in the case of A, it is determined that sufficient peel strength as a bioelectrode is maintained.

### [Example 1]

84dtex-72F polyester filaments were used to knit a smooth weave circular knitted fabric using a circular knitting machine. The obtained circular knitted fabric was scoured with a 3% by mass aqueous solution of sodium hydroxide (at 80°C for 20 minutes), washed with water (at 50°C for 10 minutes), and heat-set (at 180°C for 1 minute) using a dry heat treatment machine to obtain the fiber base material. The obtained fiber base material had a basis weight of 150 g/m². A mixed solution comprising 400 g/L of (A) "'LION PASTE (registered trademark)' W-311N" (manufactured by Lion Specialty Chemicals Co., Ltd.; Ketjenblack dispersion) comprising carbon black as a conductive material, 500 g/L of (B) "'EVAFANOL (registered trademark) HA-107C'" (manufactured by NICCA CHEMICAL Co., Ltd.; water-based urethane resin) as a binder, and 40 g/L of (C) "SN-Thickener 920" (manufactured by SAN NOPCO LIMITED; acrylate) as a water-based thickener was applied to the obtained fiber base material by a bar coater and heated at 130°C to obtain the bioelectrode. The properties of used parts and the obtained bioelectrode are shown in Table 1.

### [Example 2]

A bioelectrode was manufactured by performing the same treatment as in Example 1, except that the fiber base material was changed to a nylon jersey. For the nylon jersey, a circular knitted fabric was manufactured using 70d-24F and 78T/2-24F nylon 6 filaments for wales and courses, and the same treatment as in Example 1 was performed to obtain the fiber base material. The obtained fiber base material had a basis weight of 260 g/m². The properties of used parts and the obtained bioelectrode are shown in Table 1.

### [Example 3]

A bioelectrode was manufactured by performing the same treatment as in Example 1, except that a three-layered non-woven fabric composed of a polypropylene-based melt-blown fiber web, a spunbonded fiber web, and a melt-blown fiber web was used as the fiber base material. A basis weight of the fiber base material was 65 g/m². A water pressure resistance was 1091 mm H₂O. The properties of used parts and the obtained bioelectrode are shown in Table 1.

### [Example 4]

A bioelectrode was manufactured by the same method as in Example 3, except that the binder was changed to "'SUPERFLEX (registered trademark)' E-4800" (manufactured by DKS Co. Ltd.; polyether-based urethane resin). The properties of used parts and the obtained bioelectrode are shown in Table 1.

### [Example 5]

A bioelectrode was manufactured by the same manufacturing method as in Example 4, except that the percentage of the mixed layer was changed to 0.4%. The properties of used parts and the obtained bioelectrode are shown in Table 1.

### [Example 6]

A bioelectrode was manufactured by the same manufacturing method as in Example 1, except that the percentage of the mixed layer was changed to 57%. The properties of used parts and the obtained bioelectrode are shown in Table 1.

### [Example 7]

A bioelectrode was manufactured by the same manufacturing method as in Example 1, except that the fiber base material was changed to a polyester woven fabric and the percentage of the mixed layer was changed to 24%. For the fiber base material, 84dtex-72F polyester filaments were used to manufacture a plain weave woven fabric using a loom. The obtained woven fabric was scoured with an aqueous solution of 0.5 g/L of a surfactant and 0.7 g/L of sodium hydroxide (at 80°C for 20 minutes), washed with water (at 50°C for 10 minutes), and heat-set (at 180°C for 1 minute) using a dry heat treatment machine to obtain the fiber base material. The obtained fiber base material had a basis weight of 110 g/m². A cover factor of the obtained fiber base material was 2300. The properties of used parts and the obtained bioelectrode are shown in Table 1.

### [Comparative Example 1]

A bioelectrode was manufactured by performing the same treatment as in Example 1, except that no thickener was added. The properties of used parts and the obtained bioelectrode are shown in Table 2.

### [Comparative Example 2]

A bioelectrode was manufactured by the same manufacturing method as in Example 3, except that the binder was changed to "High Resin SD-319" (Takamatsu Oil & Fat Co., Ltd.; silicone resin). The properties of used parts and the obtained bioelectrode are shown in Table 2.

### [Comparative Example 3]

A bioelectrode was manufactured by the same manufacturing method as in Example 3, except that the binder was changed to "'KM-9772'" (manufactured by Shin-Etsu Chemical Co., Ltd.; silicone rubber). The properties of used parts and the obtained bioelectrode are shown in Table 2.

### [Table 1]

**Table 1**

| | | Example 1 | Example 2 | Example 3 | Example 4 | Example 5 | Example 6 | Example 7 |
|---|---|---|---|---|---|---|---|---|
| Used parts | Material of fiber base layer | PET | Ny | PP | PP | PP | PET | PET |
| | Form | Knitted fabric | Knitted fabric | Non-woven fabric | Non-woven fabric | Non-woven fabric | Knitted fabric | Woven fabric |
| | Binder | PC-based urethane | PC-based urethane | PC-based urethane | Ether-based urethane | Ether-based urethane | PC-based urethane | PC-based urethane |
| | Water-based thickener | Present | Present | Present | Present | Present | Present | Present |
| Surface resistance of fiber base material (Ω) | | > 9.9 × 10¹² | 6.9 × 10¹² | > 9.9 × 10¹² | > 9.9 × 10¹² | > 9.9 × 10¹² | > 9.9 × 10¹² | > 9.9 × 10¹² |
| Layer thickness (um) | Conductive layer | 65 | 50 | 55 | 47 | 51 | 24 | 37 |
| | Mixed layer | 59 | 550 | 45 | 48 | 2 | 266 | 41 |
| | Fiber base layer | 456 | 600 | 290 | 315 | 448 | 180 | 93 |
| Percentage of mixed layer (%) | | 10 | 46 | 12 | 12 | 0.4 | 57 | 24 |
| Resistance value of bioelectrode (Q) | | 47 | 97 | 119 | 123 | 98 | 205 | 64 |
| Volume resistivity of bioelectrode (Ω·cm) | | 2 | 23 | 5 | 5 | 2 | 24 | 2 |
| Static friction resistance of bioelectrode (N/cm²) | | 0.25 | 0.22 | 0.33 | 0.26 | 0.27 | 0.21 | 0.25 |
| Rate of bending resistance change of bioelectrode (times) | | 3.4 | 1.7 | 1.1 | 1.1 | 1.0 | 4.5 | 2.1 |
| Peel strength of conductive layer (cN/cm) | | A | A | A | A | B | A | A |

| | | | | | | | | |
|---|---|---|---|---|---|---|---|---|
| PET: polyethylene terephthalate Ny: nylon PP: polypropylene PC: polycarbonate | | | | | | | | |

### [Table 2]

**Table 2**

| | | Comparative Example 1 | Comparative Example 2 | Comparative Example 3 |
|---|---|---|---|---|
| Used parts | Material of fiber base layer | PET | PP | PP |
| | Form | Knitted fabric | Non-woven fabric | Non-woven fabric |
| | Binder | PC-based urethane | Silicone resin | Silicone rubber |
| | Water-based thickener | Not present | Present | Present |
| Surface resistance of fiber base material (Ω) | | > 9.9 × 10¹² | > 9.9 × 10¹² | > 9.9 × 10¹² |
| Layer thickness (um) | Conductive layer | 0 | 37 | 53 |
| | Mixed layer | 495 | 22 | 38 |
| | Fiber base layer | 0 | 334 | 301 |
| Percentage of mixed layer (%) | | 100 | 6 | 10 |
| Resistance value of bioelectrode (Q) | | 230 | 137 | 126 |
| Volume resistivity of bioelectrode (Ω·cm) | | 45 | 3 | 5 |
| Static friction resistance of bioelectrode (N/cm²) | | 0.2 | 0.15 | 0.11 |
| Rate of bending resistance change of bioelectrode (times) | | 6.1 | 1.1 | 1.1 |
| Peel strength of conductive layer (cN/cm) | | A | A | A |

| | | | | |
|---|---|---|---|---|
| PET: polyethylene terephthalate Ny: nylon PP: polypropylene PC: polycarbonate | | | | |

In the bioelectrodes of the examples, since the water-based thickener is added, the percentage of the mixed layer in the electrode is low, and excellent volume resistivity is accomplished. Accordingly, they are considered to have high sensitivity in biosignal acquisition. In addition, since the static friction resistance is very high, it is expected that the bioelectrodes are prevented from moving out of position when worn and the generation of artifacts when acquiring biosignals is prevented. Further, since a non-conductive fiber base material is used and a flexible urethane resin is used as a binder, they have excellent comfortability to wear. On the other hand, since no thickener is added in Comparative Example 1, the percentage of the mixed layer in the electrode is high and the volume resistivity is high, and there is a concern that biosignal acquisition may be reduced when compared with the examples. In addition, since no urethane-based binder is used in Comparative Examples 2 and 3, the static friction resistance is low and the bioelectrode may move out of position when worn, and there is a concern that artifacts may be generated when acquiring biosignals.

### INDUSTRIAL APPLICABILITY

The bioelectrode of the present invention is suitably used as a bioelectrode for measuring bioelectric signals such as brain waves, electrocardiograms, or electromyograms of human bodies or animals, or for applying electric stimulation to a living body.

### DESCRIPTION OF REFERENCE SIGNS

1: Conductive material
2: Fiber base material
3: Conductive layer
4: Mixed layer
5: Fiber base layer

## Claims

1. A bioelectrode comprising a layered structure of a fiber base layer composed of non-conductive fibers and a conductive layer, wherein the conductive layer is formed of a conductive material comprising carbon black, urethane resin, and a water-based thickener.

2. The bioelectrode according to claim 1, further comprising a mixed layer in which fibers and a conductive material are mixed located between the fiber base layer and the conductive layer.

3. The bioelectrode according to claim 2, wherein a percentage of the mixed layer, which is a value obtained by dividing a thickness value of the mixed layer by a total value of each thickness of the conductive layer, the mixed layer, and the fiber base layer provided in the bioelectrode and multiplying a resultant value by 100, is 0.1 to 80%, and a thickness of the conductive layer is 1 to 200 µm.

4. The bioelectrode according to any one of claims 1 to 3, wherein the water-based thickener is a polyacrylic acid-based compound.

5. The bioelectrode according to any one of claims 1 to 4, wherein the urethane resin includes at least one selected from a group consisting of an ether-based urethane resin and a carbonate-based urethane resin.

6. The bioelectrode according to any one of claims 1 to 5, wherein a volume resistivity of the bioelectrode is 1 × 10⁶ Ω·cm or less.

7. The bioelectrode according to any one of claims 1 to 6, wherein a surface resistance value of the fiber base layer is 1 × 10¹⁰ Ω or more.
